# FASCICULE DE BREVET EUROPEEN

(11) **EP 2 297 328 B1**
(45) Date de publication et mention de la délivrance du brevet: **22.03.2017**
(21) Numéro de dépôt: 09757695.3
(22) Date de dépôt: 03.06.2009
(51) Int. Cl.: C12P 7/26, A61K 8/35, A61Q 13/00, A23L 27/24, A23L 27/29, C11B 9/00

(54) **Procédé de production de la 9-décen-2-one naturelle par bioconversion de l'acide undécylénique a l'aide d'une moisissure**
Verfahren zur Herstellung von natürlichem 9-Decen-2-on durch Biokonvertierung von Undecylensäure mit einem Schimmelpilz
Method for producing natural 9-decen-2-one by bioconverting undecylenic acid using a mold

(30) Priorité: 03.06.2008 FR 0853672
(43) Date de publication de la demande: 23.03.2011
(73) Titulaire: V. Mane Fils, 06620 Bar sur Loup (FR)
(72) Inventeur: MANE, Jean, F-06130 Grasse (FR); ZUCCA, Joseph, F-06130 Grasse (FR)
(74) Mandataire: Gevers & Orès
(86) Numéro de dépôt international: PCT/FR2009/000646
(87) Numéro de publication internationale: WO 2009/147319

(56) Documents cités:
- EP-A- 0 312 746
- EP-A- 0 390 624
- FR-A- 2 877 339
- DATABASE WPI Week 198415 Thomson Scientific, London, GB; AN 1984-092349 XP002524503 & JP 59 039822 A (MEIJI MILK PROD CO LTD) 5 mars 1984 (1984-03-05)
- DATABASE WPI Week 199227 Thomson Scientific, London, GB; AN 1992-223243 XP002524501 & JP 04 148688 A (SHOWA SANGYO CO) 21 mai 1992 (1992-05-21) -& DATABASE CA [Online] CHEMICAL ABSTRACTS SERVICE, COLUMBUS, OHIO, US; 1 novembre 1992 (1992-11-01), XP002524499 Database accession no. 117:169602
- DATABASE WPI Week 199049 Thomson Scientific, London, GB; AN 1990-366323 XP002524502 & JP 02 265495 A (SHOWA SANGYO CO) 30 octobre 1990 (1990-10-30) -& DATABASE CA [Online] CHEMICAL ABSTRACTS SERVICE, COLUMBUS, OHIO, US; 31 mai 1991 (1991-05-31), XP002524500 Database accession no. 114:205568
- FORNEY F W ET AL: "The biology of methyl ketones." JOURNAL OF LIPID RESEARCH JUL 1971, vol. 12, no. 4, juillet 1971 (1971-07), pages 383-395, XP002524498 ISSN: 0022-2275

## Description

La présente invention porte sur la bioconversion, à l'aide d'une moisissure, de l'acide undécylénique en 9-décen-2-one, cette dernière pouvant être utilisée dans des applications de parfumerie et d'aromatique alimentaire.

La société Demanderesse a mis en évidence que la 9-Décen-2-one (ou Décénone-2 ou Méthyl Octènyl Cétone, RN CAS 35194-30-0) est une méthyl-cétone présentant des propriétés olfactives et gustatives telles qu'elle peut être utilisée dans toutes les applications de l'industrie aromatique (arômes et fragrances). En effet, elle présente une note verte et grasse ainsi que des notes fruitées, notamment de poire, pomme, ananas, fruit de la passion et fruit exotique en général.

Cette cétone, tout comme les autres méthyl-cétones, est formée par une voie métabolique qui emprunte les premières étapes de la β-oxydation après oxydation de l'acide gras. Cette voie métabolique pour la formation des méthyl-cétones est utilisée par les mycètes lors de la détoxification des acides gras.

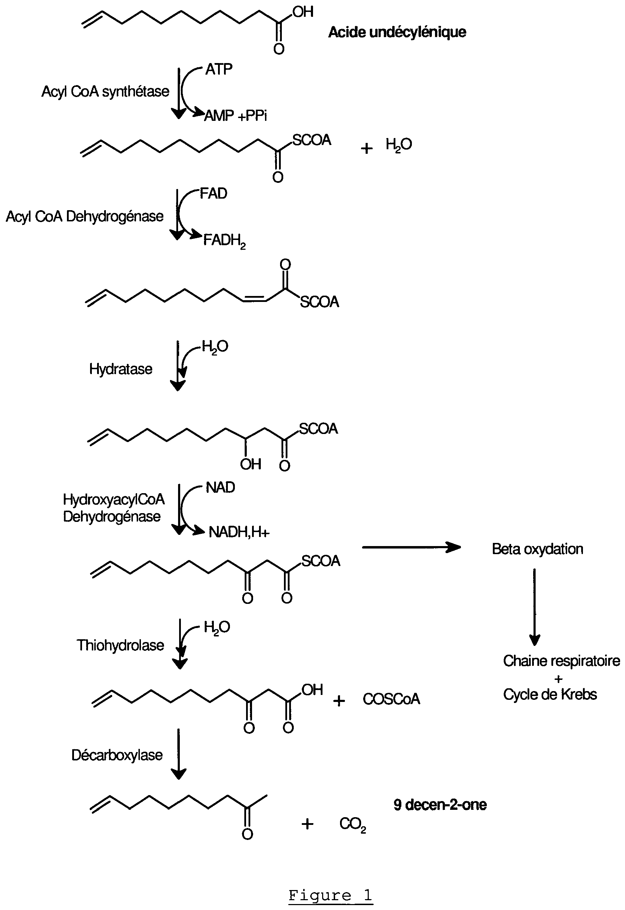

Comme il peut être observé par la représentation de la voie métabolique (Figure 1), le substrat utilisé au cours de la bioconversion est l'acide undécylénique qui est obtenu par le cracking (opération physique transformant le produit brut en composés plus fins) de l'acide ricinoléique issue de l'huile de ricin puis purifié par une distillation sèche. Enfin, à la suite d'une étape de décarboxylation, la méthyl-cétone obtenue en bout de parcours possède un carbone de moins que l'acide gras d'origine.

A la connaissance de la Société déposante, il n'existe aucun procédé biotechnologique aujourd'hui qui décrit et/ou permet la synthèse de la 9-décen-2-one. En effet cette molécule ne peut être obtenue à ce jour que par synthèse chimique et ceci avec des rendements médiocres. Par ailleurs, les arômes de synthèse présentent l'inconvénient d'être moins appréciés par les consommateurs que les arômes naturels. Par conséquent, la présente invention a pour but de fournir un procédé alternatif d'obtention de molécules aromatiques naturelles, en particulier celles obtenues par le biais de la 9-décen-2-one, par des procédés biologiques, notamment de bioconversion, mettant en oeuvre des microorganismes tels que les moisissures.

Au sens de la présente invention, on entend par « bioconversion » la transformation biologique d'un substrat, de préférence issu d'une source naturelle, pour obtenir des substances aromatisantes naturelles qui peuvent être formulées dans des arômes ou des fragrances qualifiés de naturels.

De façon surprenante et inattendue, la Société déposante a trouvé qu'il était possible de satisfaire à cet objectif grâce à un procédé de production de la 9-décen-2-one, caractérisé par la bioconversion de l'acide undécylénique à l'aide d'une moisissure, ledit procédé comprenant les étapes suivantes:
a) la culture de ladite moisissure;
b) l'ajout d'acide undécylénique comme substrat de bioconversion, en présence d'huile;
c) la bioconversion du substrat en 9-décen-2-one,
d) l'extraction et la purification de la 9-décan-2-one.
Ainsi, la présente invention concerne un procédé de production de la 9-décen-2-one, caractérisé par la bioconversion de l'acide undécylénique à l'aide d'une moisissure appartenant à la famille des Mortierellaceae ou à la famille des Mucoraceae, de préférence au genre *mortierella,* au genre *Mucor* ou au genre *Rhizopus,* ledit procédé comprenant les étapes suivantes:
a) la culture de ladite moisissure;
b) l'ajout du substrat acide undécylenique avec un débit de 0,1 à 0,9 g/L/h en présence d'huile;
c) la bioconversion du substrat en 9-décen-2-one,
d) l'extraction et la purification de la 9-décen-2-one.
Au sens de la présente invention, le terme « moisissure » correspond à des champignons microscopiques filamenteux uniou multicellulaires. Parmi les moisissures, on distingue les Phycomycètes et les Septomycètes. Au sein des Phycomycètes, on trouve le phylum des Oomycotinées et le phylum des Zygomycotinées. Au sein des Septomycètes, on trouve le phylum des Ascomycotinées, le phylum des Basidiomycotinées et le phylum des Deutéromycotinées.
Dans un mode de réalisation de la divulgation, la moisissure selon la divulgation appartient au phylum des Deutéromycotinées, Classe des Hyphomycètes, Ordre des Tuberculariales, Famille des Aspergillaceae. Plus préférablement, ladite moisissure appartient au genre Aspergillus ou Pénicillium. Dans un premier mode de réalisation préféré, ladite moisissure appartient à l'espèce *Aspergillus oryzae* ou à l'espèce *Aspergillus niger.* Dans un second mode de réalisation préféré, ladite moisissure appartient à l'espèce *Pénicillium roquefortii* ou à l'espèce *Pénicillium camenbertii.*

Parmi les moisissures *Aspergillus oryzae,* on peut citer les souches de collection suivantes : *Aspergillus oryzae* DSMZ 1861, *Aspergillus oryzae* DSMZ 1864, *Aspergillus oryzae* DSMZ 1147, *Aspergillus oryzae* DSMZ 63303, *Aspergillus oryzae* CBS 570.65, *Aspergillus oryzae* CBS 819.72, *Aspergillus oryzae* CBS 110.27.
Parmi les moisissures *Aspergillus niger,* on peut citer les souches de collection suivantes : *Aspergillus niger* DSMZ 823, *Aspergillus niger* DSMZ 2466.
Parmi les moisissures *Penicillium roquefortii,* on peut citer les souches de collection suivantes : *Pénicillium roquefortii* CBS 221-30, *Pénicillium roquefortii* PRB 18, *Pénicillium roquefortii* DSMZ 1079, *Penicillium roquefortii* DSMZ 1080. Parmi les moisissures *Penicillium camembertii,* on peut citer la souche *Penicillium camembertii* DSMZ 1233. La moisissure selon l'invention appartient au phylum des Zygomycotinées, Classe des Zygomycètes, Ordre des Mucorales, Famille des Mortierellaceae ou Famille des Mucoraceae.
Plus préférablement, ladite moisissure appartient au genre Mortierella ou au genre Mucor ou au genre Rhizopus, et encore plus préférablement, ladite moisissure appartient à l'espèce *Mortierella isabellina ou* Mortierella rammniana ou à l'espèce *Mucor racemosus ou Mucor miehei ou à l'espèce Rhizopus arrhizus* ou *Rhizopus oryzae.*
Parmi les moisissures appartenant au genre Mortierella, on peut citer les souches de collection suivantes : *Mortierella isabellina* DSMZ 1414, *Mortierella isabellina* CBS 100559, *Mortierella isabellina* CBS 221.29, *Mortierella isabellina* CBS 194.28, *Mortierella isabellina* CBS 208.32, *Mortierella isabellina* CBS 224.35, , *Mortierella isabellina* CBS 560.63, *Mortierella isabellina* CBS 167.80, *Mortierella isabellina* CBS 493.83, *Mortierella isabellina* CBS 309.93, *Mortierella isabellina* CBS 250.95, *Mortierella isabellina* CBS 109075, *Mortierella ramanniana* CBS 112.08, *Mortierella ramanniana* CBS 219.47, *Mortierella ramanniana* CBS 243.58, *Mortierella ramanniana* CBS 478.63, *Mortierella ramanniana* CBS 852.72, *Mortierella ramanniana* CBS 366.95, *Mortierella ramanniana* CBS 101226.
Parmi les moisissures appartenant au genre Mucor, on peut citer les souches de collection suivantes : *Mucor javanicus* DSMZ 1222, *Mucor racemosus* DSMZ 62760, *Mucor rouxii* DSMZ 1191.
Parmi les moisissures appartenant au genre Rhizopus, on peut citer les souches de collection suivantes : *Rhizopus arrhizus oryzae* DSMZ 905, *Rhizopus oryzae* DSMZ 2199, *Rhizopus delemar* DSMZ 853, Rhizopus niveus DSMZ 2194, *Rhizomucor miehei* DSMZ 1330.

La culture visée à l'étape a) du procédé selon l'invention, comprend la réalisation d'une culture, de préférence semi-concentrée, par exemple par amplification cellulaire, dans un milieu de culture approprié. Cette culture est précédée par une pré-culture de la souche dans un premier milieu de culture plus adapté aux premières étapes de multiplication.
Dans un mode de réalisation préféré de l'invention, le temps de culture de ladite moisissure varie entre 20 et 30 heures et dépend en fait de l'état de croissance du mycélium, ce dernier pouvant être observé au microscope. Il est en effet préféré que la biomasse minimale de la moisissure soit comprise entre 7 et 15 g/l et plus préférablement entre 9 et 12 g/l, avant de procéder à la bioconversion selon l'invention.

L'étape b) du procédé consiste à ajouter le substrat à la culture cellulaire. Selon l'invention, la synthèse par voie biologique de la 9-décen-2-one fait intervenir le substrat le plus approprié qui est l'acide undécylénique . Selon une alternative (non selon l'invention), le substrat est l'un des esters de l'acide undécyclénique, l'ester méthylique ou l'ester éthylique de l'acide undécylénique. Il va de soi que le substrat peut être un mélange de différents substrats appropriés, en particulier un mélange d'acide undécylénique et d'un ou plusieurs de ses esters.

Selon l'invention, ledit acide undécylénique est ajouté au milieu de fermentation avec un débit de 0,1 à 0,9 g/L/h, de préférence de 0,2 à 0,7 g/L/h et très préférentiellement de 0,3 à 0,55 g/L/h.

L'étape c) du procédé consiste en la bioconversion de l'acide undécylénique en 9-décen-2-one. La durée de cette phase de bioconversion est généralement de 36 à 96 heures.

En raison de la grande toxicité de l'acide undécylénique pour les mycètes, une période d'adaptation peut s'avérer nécessaire afin de préparer la moisissure à convertir le substrat. Ainsi, selon un mode de réalisation préféré, l'étape de bioconversion du procédé selon l'invention comprend une première phase d'adaptation de la moisissure dans laquelle ledit acide undécylénique est ajouté à un débit de 0,1 à 0,5 g/L/h, puis une seconde phase dans laquelle l'acide undécylénique est ajouté à un débit de 0,25 à 0,9 g/l/h. De façon avantageuse, la durée de ladite phase d'adaptation est inférieure à 20 heures, de préférence inférieure à 12 heures et plus préférentiellement inférieure à 6 heures.

Selon un mode de réalisation préféré, l'acide undécylénique est ajouté en présence d'huile. Ladite huile peut être choisie dans le groupe comprenant les huiles classiques alimentaires telles que les huiles de soja, maïs, tournesol, ou autre ou des triglycérides contenant des acides gras à chaînes courtes tels que le Miglyol, ou encore des huiles blanches telles que l'huile de paraffine ou des huiles minérales composées d'hydrocarbures à longue chaine. De manière préférée, ladite huile est l'huile de tournesol partiellement hydrogénée ou riche en acide oléique
De manière préférée, ladite huile est ajoutée dans les proportions suivantes par rapport au substrat : Acide undécylénique/Huile = 1/4 acide undécylénique - 3/4 huile ou 1/3 acide undécylénique - 2/3 huile ou 1/2 acide undécylénique - 1/2 huile.
De façon avantageuse, ladite huile est ajoutée au milieu de fermentation à un débit de 0,4 à 4,0 g/L/h, de préférence de 0,5 à 3 g/L/h et très préférentiellement de 0,75 à 2,5 g/L/h.

Comme tous types de microorganismes en situation de bioconversion forcée, les mycètes ont besoin d'une source de carbone permettant de couvrir leurs besoins énergétiques comme par exemple une source de glucide, de préférence de glucose ou de maltose. L'apport de glucose au cours de la bioconversion est préféré. Par conséquent, selon un autre mode de réalisation préféré de l'invention, du glucose ou du maltose est ajouté simultanément à l'acide undécylénique et à l'huile. De façon avantageuse, ledit glucose ou ledit maltose est ajouté au milieu de fermentation à un débit inférieur à 1 g/L/h, de préférence inférieur à 0,75 g/L/h et très préférentiellement inférieur à 0,5 g/L/h.

Selon un autre mode de réalisation préféré de l'invention, l'ajout dudit acide undécylénique, de ladite huile et optionnellement dudit glucose ou maltose dans le milieu de fermentation se fait en continu pendant 5 à 96 heures, de préférence pendant 24 à 72 heures, et plus préférablement pendant 24 à 48 heures

Selon un autre mode de réalisation préféré, la bioconversion selon l'invention est réalisée à une température comprise entre 25°C et 35°C, de préférence entre 27 à 30°C.

Selon un autre mode de réalisation préféré, le pH du milieu de fermentation selon l'invention est de 5 à 8, de préférence de 5,5 à 7,5, et plus préférentiellement de 6 à 7. Dans le cadre de l'invention, le contrôle du pH se fait à l'aide de soude (par exemple NaOH 5N stérile ou autre base classique en fermentation (NH4OH, KOH, et..)).

Au cours de la bioconversion, il est important d'avoir une bonne agitation ainsi qu'une bonne aération du milieu de fermentation. Dans le fermenteur, l'agitation peut se créer grâce à plusieurs pales d'agitation. De façon avantageuse et selon la taille du fermenteur, par exemple pour un fermenteur de 6 litres, l'agitation adéquate nécessaire selon l'invention est de 200 à 1200 rpm, et de préférence de 300 à 900 rpm. L'aération, quant à elle, peut se faire par injection d'air au niveau des pales d'agitation. De façon avantageuse, l'aération adéquate du milieu de fermentation selon l'invention est inférieure ou égale à 1 vvm, de préférence comprise entre 0,2 et 0,8 vvm et plus préférentiellement comprise entre 0,3 et 0,7 vvm.

Dans un autre mode de réalisation préféré, la bioconversion selon l'invention est arrêtée par ajout dans le milieu de culture, d'un acide. Ledit acide peut être choisi dans le groupe comprenant l'acide phosphorique, l'acide sulfurique, l'acide chlorhydrique ou l'acide citrique. De préférence, ledit acide est l'acide phosphorique ou l'acide citrique.

Dans l'étape d), l'extraction de la 9-décen-2-one peut être réalisée par hydrodistillation ou par extraction à l'aide d'un solvant choisi dans le groupe comprenant le cyclohexane, le Methyl Cyclohexane, ou l'acétate d'éthyle. De préférence, le solvant utilisé est le Cyclohexane ou un mélange Cyclohexane-Acétate d'éthyle.
La purification de la 9-décen-2-one peut ensuite être réalisée par dérésinage puis distillation fractionnée.
Ce procédé d'extraction/purification permet ainsi d'obtenir une molécule pure à plus de 98% avec un rendement d'extraction de l'ordre de 75 à 85%.
Le rendement de bioconversion en 9-décen-2-one par rapport au substrat employé, l'acide undécylènique, est d'environ 25 à 35%.

La divulgation concerne également la 9-décen-2-one obtenue par le procédé tel que décrit ci-dessus.

La divulgation a pour objet l'utilisation de la 9-décen-2-one (CAS 35194-30-0) dans toutes les applications de parfumerie et d'aromatique alimentaire, en particulier pour la fabrication de parfums, de matières odorantes, de compositions cosmétiques ou alimentaires, ou comme additif alimentaire.

Dans un mode de réalisation préféré, la divulgation a pour objet l'utilisation de la 9-décen-2-one obtenue par le procédé tel que décrit ci-dessus dans toutes les applications de parfumerie et d'aromatique alimentaire, en particulier pour la fabrication de parfums, de matières odorantes, de compositions cosmétiques ou alimentaires, ou comme additif alimentaire.

Au sens de la présente divulgation, le terme parfumerie désigne non seulement la parfumerie au sens habituel du terme, mais également les autres domaines dans lesquels l'odeur des produits est importante. Il peut s'agir, sans que cela soit limitatif, de compositions de parfumerie au sens habituel du terme, telles que bases et concentrés parfumants, eaux de Cologne, eaux de toilette, parfums et produits similaires ; de compositions topiques - en particulier cosmétiques - telles que crèmes pour le visage et le corps, poudres de talc, huiles pour cheveux, shampoings, lotions capillaires, onguents, sels et huiles de bain, gels de douche et de bain, savons de toilette, anti-transpirants et désodorisants corporels, lotions et crèmes de rasage, savons, crèmes, dentifrices, bains de bouche, pommades, et produits similaires ; et de produits d'entretien, tels qu'assouplissants, détergents, lessives, désodorisants d'ambiance, aérosols et produits similaires.

Le terme odorant est utilisé pour désigner un composé qui exhale une odeur.

Par aromatique alimentaire, on entend toute utilisation des composés de la divulgation pour l'aromatisation de toute denrée alimentaire liquide ou solide, humaine ou animale notamment des boissons, des produits laitiers, des crèmes glacées, des bonbons, des chewing-gums etc... mais aussi pour l'aromatisation des tabacs.

La 9-decen-2-one est ainsi utilisable en compositions parfumantes ou en compositions aromatiques alimentaires pour contribuer à donner des notes exotiques, florales ou fruitées, en particulier ananas, poire, fruit de la passion, fruits exotiques. Selon les applications, on utilisera cette molécule de 9-décen-2-one dans des proportions déterminées par l'homme de l'art.

De préférence, la 9-decen-2-one sera utilisée dans des quantités comprises entre 0,00025% et 30%, préférablement entre 0,0025 et 20%, plus préférablement entre 0,025 et 10%, en poids par rapport au poids total de la composition dans laquelle elle est présente. Elle pourra entrer dans la composition de solides ou de liquides et notamment dans la composition de gels, crèmes, pommades et/ou sprays.

La 9-decen-2-one pourra également être utilisée dans une composition elle-même odorante, ou dans une composition dans laquelle l'agent odorant est utilisé pour masquer ou neutraliser certaines odeurs.

La Figure 1 décrit la bioconversion de l'acide undécylénique en 9-décen-2-one.

### EXEMPLES

### Exemple 1 :

On ensemence les souches Mortierella isabellina (A), Aspergillus oryzae (B) et Penicillium roquefortii (C) [origine = tube congelé à - 80°C] sur gélose au malt, et on incube à 27°C (pour A) ou 30°C (pour B et C) pendant 72 heures.
On ensemence la préculture précédente dans 3,5 l de milieu au malt en fermenteur de 6 l :

| | |
|---|---|
| Extrait de malt : | 50 g/l |
| Extrait de levure : | 7,5 g/l |
| pH | 6,5 |

### Mortierella isabellina (A)

On incube pendant 24 heures ladite souche à 27°C, l'agitation est de 500 rpm et l'aération est de 0.3 vvm.

### Aspergillus oryzae (B)

On incube pendant 24 heures ladite souche à 30°C, l'agitation est de 600 rpm et l'aération de 0,5 vvm.

### Penicillium roquefortii (C)

On incube pendant 24 heures ladite souche à 30°C, l'agitation est de 600 rpm et l'aération de 0,3vvm.
Le poids sec de mycélium à la fin de la culture pour chaque souche est d'environ 9 à 11 g/l.
L'acide undécylénique est ensuite distribué avec un débit de 0,15 g/l/h pendant 6 h puis au débit de 0,33 g/l/h pendant 72 h : soit un total de 25 g/l. Cet acide undécylénique est distribué en mélange avec de l'huile de tournesol hydrogénée (1/3 acide-2/3 huile) ; cette huile est donc distribuée aux débits de 0,6 g/l/h puis de 1 g/l/h. On distribue parallèlement du glucose en continu au débit de 0,36 g/l/h pendant 72 h. On régule le pH à 6,5 pendant toute la durée de la conversion avec NaOH 5 N. On augmente la vitesse à 900 rpm et on aère au débit de 1 vvm pour Mortierella isabellina et 0,3 vvm pour Aspergillus oryzae ou Penicillium roquefortii. On poursuit la conversion pendant 72 heures.

En conditions non optimisées, on obtient une production comprise entre 5 et 7 g/l de 9-décen-2-one.

### Exemple 2 : Conversion avec Aspergillus oryzae (B)

On ensemence Aspergillus oryzae [origine = tube congelé à - 80°C] sur gélose au malt, et on incube à 30°C pendant 72 heures.

On ensemence la préculture précédente dans 3,5 l de milieu au malt (même concentration que dans l'exemple 1).

On incube à 30°C, 600 rpm, 0,5 vvm d'air, pH libre, pendant 24 heures. On obtient un poids sec de 11 g/l.

L'acide undécylénique est ensuite distribué à un débit de 0,5 g/l/h pendant 6 H puis au débit de 0,9 g/l/h pendant 48 h : soit un total de 46 g/l. Cet acide undécylénique est distribué en mélange avec de l'huile de tournesol hydrogénée (1/4 acide-3/4 huile) ; cette huile est donc distribuée aux débits de 1,5 g/l/h puis de 2,7 g/l/h. On distribue parallèlement du glucose en continu au débit de 0,36 g/l/h pendant 48 h. On régule le pH à 6 pendant toute la durée de la conversion avec NaOH 5 N. On augmente la vitesse à 900 rpm et on aère au débit de 0,3 vvm. On poursuit la conversion pendant 48 heures.

On obtient une production de 16,5 g/l de 9-décen-2-one, soit un rendement de bioconversion de 35%.

### Exemple 3 : Conversion avec Mortierella isabellina (A)

On ensemence Mortierella isabellina [origine = tube congelé à - 80°C] sur gélose au malt, et on incube à 27°C pendant 72 heures.

On ensemence la préculture précédente dans 3,5 l de milieu au malt (même concentration que dans l'exemple 1).

On incube pendant 24 heures à 27°C, l'agitation est de 600 rpm et l'aération est de 0,5 vvm. On obtient un poids sec de 11,3 g/l.

L'acide undécylénique, est ensuite distribué à un débit de 0,27 g/l/h pendant 6 H puis au débit de 0,477 g/l/h pendant 48 h : soit un total de 46 g/l. Cet acide undécylénique est distribué en mélange avec de l'huile de tournesol hydrogénée (1/4 acide-3/4 huile) ; cette huile est donc distribuée aux débits de 0,8 g/l/h puis de 1,43 g/l/h. On distribue parallèlement du glucose en continu au débit de 0,32 g/l/h pendant 48 h. On régule le pH à 7,5 pendant toute la durée de la conversion avec NaOH 5 N. On augmente la vitesse à 900 rpm et on aère au débit de 1 vvm. On poursuit la conversion pendant 48 heures.

On obtient une production de 8 g/l de 9-décen-2-one, soit un rendement de bioconversion de 33%.

### Exemple 4 : Conversion avec Penicillium roquefortii (C)

On ensemence Penicillium roquefortii [origine = tube congelé à - 80°C] sur gélose au malt, et on incube à 30°C pendant 72 heures.

On ensemence la préculture précédente dans 3,5 l de milieu au malt (même concentration que dans l'exemple 1).

On incube pendant 22 heures à 30°C, l'agitation est de 600 rpm et l'aération est de 0,3 vvm. On obtient un poids sec de 10 g/l.

L'acide undécylénique est ensuite redistribué à un débit de 0,25 g/l/h pendant 6 H puis au débit de 0,45 g/l/h pendant 48 h puis au débit de 0,25 g/l/h pendant 24 H : soit un total d'environ 30 g/l. Cet acide undécylénique est distribué en mélange avec de l'huile de tournesol hydrogénée (1/3 acide-2/3 huile) ; cette huile est donc distribuée aux débits de 0,5 g/l/h puis de 0,9 g/l/h puis 0,5 g/l/h. On distribue parallèlement du glucose en continu au débit de 0,36 g/l/h pendant 72 h. On régule le pH à 6,5 pendant toute la durée de la conversion avec NaOH 5 N. On augmente la vitesse à 900 rpm et on aère au débit de 0,6 vvm. On poursuit la conversion pendant 48 heures.

On obtient une production de 10,5 g/l de 9-décen-2-one, soit un rendement de bioconversion de 35%.

### Exemple 5 : extraction - purification

On acidifie à pH 1,5 à 2 avec de l'acide phosphorique à 85 %. On ajoute le solvant d'extraction cyclohexane et on agite 1 heure à température ambiante. On centrifuge et on récupère la phase organique. On dose la méthyl cétone. On concentre le solvant et on obtient ainsi un "brut" huileux. On distille sous vide. On obtient la lactone "dérésinée" et une huile épuisée. On purifie ensuite en fractionnant la molécule sous vide. On obtient un produit pur à plus de 99 %

### Exemple 6 : Evaluation de la 9-décen-2-one en parfumerie

La 9-décen-2-one pure à 99 % a été testée sur mouillette et en solution (à 5 % dans l'éthanol) : la tête est puissante, homogène, présentant des notes rosées, aldéhydées, d'acétate de citronellyle, de feuille de coriandre et des notes marines. Le fond présente des notes de poire.

### Exemple 7 : Evaluation en aromatique alimentaire

La 9-décen-2-one pure à 99 % a été testée à 8 ppm dans l'eau minérale : elle présente des notes fruitées : poire, pomme et ananas et des notes vertes et grasses.

Elle a été jugée intéressante pour sophistiquer les formulations de fruits exotiques naturelles d'autant plus qu'il n'existe pas beaucoup de molécules naturelles disponibles pour atteindre ce but. En particulier la tonalité « Ananas » de la 9-décen-2-one constitue une alternative très attractive pour remplacer le caproate d'allyle, qui est la molécule la plus utilisée actuellement en formulation pour obtenir une tonalité de type ananas.

### Exemple 8 : Utilisation en formulation en aromatique alimentaire et en parfumerie.

Dans un arôme de fruits exotiques, l'ajout de 9-décen-2-one permet d'obtenir une tonalité typiquement « ananas ». Dans la formule suivante le remplacement poids par poids du caproate d'allyle, habituellement utilisé en formulation, par la 9-décen-2-one permet de compenser de manière très avantageuse la tonalité « ananas » conférée par le caproate d'allyle.

| | |
|---|---|
| Ethyle caproate naturel | 50g, |
| Maltol naturel | 20 g, |
| Isoamyle acétate naturel | 35 g, |
| Ethyle isovalérate naturel | 37 g, |
| menthol codex naturel | 8 g, |
| Ethyle méthyl-2-butyrate naturel | 75 g, |
| Ethyle acétate naturel | 50g, |
| Isoamyle butyrate naturel | 12 g, |
| Ethyle butyrate naturel | 35 g, |
| Camphre raffiné | 2 g, |
| 9-décen-2-one naturelle | 200 g, |
| Alcool éthylique naturel | qsp 1000 g |

L'emploi à 50ppm de ce mélange dans une boisson plate avec jus lui confère des notes "ananas" très caractéristiques ; idem lorsqu'on utilise ce mélange à 200 ppm pour typer « ananas » un sorbet.
Toujours en formulation d'arôme alimentaire naturel, la 9-décen-2-one permet d'obtenir des notes « poire » intéressantes, par exemple dans la formule suivante :

| | |
|---|---|
| Acide caproique naturel | 1 à 10 g, |
| Isoamylique alcool naturel | 20 à 60 g, |
| Hexylique alcool naturel | 10 à 50 g, |
| Ethyle caprate naturel | 20 à 80 g, |
| Ethyle caprylate naturel | 50 à 150 g, |
| Isoamyle acétate naturel | 50 à 150 g, |
| Propyle acétate naturel | 50 à 150 g, |
| Hexyle acétate naturel | 50 à 150 g, |
| Butyle acétate naturel | 50 à 150 g, |
| Ethyle acétate naturel | 50 à 150g, |
| 9-décen-2-one naturelle | 20 à 50 g, |
| Propylène Glycol | qsp 1000 g |

L'emploi à 100 ppm de ce mélange dans une boisson faiblement sucrée à 5% et légèrement acidulée (0,1% acide citrique), lui confère des notes "poire" très caractéristiques même si la dose de 9-décen-2-one est très faible (2 à 5 ppm).

En formulation en parfumerie, la 9-décen-2-one permet de pousser le côté « fleuri, vert, violette » ainsi que la note « aldéhydée fruitée » d'un shampoing, par exemple dans la formule suivante dans laquelle la 9-décen-2-one est utilisée à 1%, ce qui rend la note parfumée du shampoing plus complexe et plus ronde :

| | |
|---|---|
| Benzyle Acétate | 70g, |
| Alcool Phényléthylique | 100g, |
| Gamma undécalactone | 4g, |
| Ambrettolide cis-iso | 10g, |
| Aldéhyde Anisique | 25g, |
| Bacdanol | 80g, |
| Citronellol | 85g, |
| Ethyl Vanilline | 2g, |
| Geraniol | 40g, |
| Methyl Dihydro Jasmonate | 120g, |
| Oxacyclohexadecen-2-one | 100g, |
| Heliotropine | 20g, |
| 2-acetyl-1,2,3,4,5,6,7,8-octahydro-2,3,8,8-tetramethyl naphtalene | 110g, |
| Isoeugenol | 2g, |
| 4-(1,1-dimethylethyl)-<a>-methyl-benzenepropanal | 110g, |
| Linalool | 60g, |
| Methyl ionone | 30g, |
| Vanilline | 10g, |
| 2-octynoic acid, methyl ester | 12g, |
| 9-décen-2-one | 10g, |
| Total | 1000 g. |

Dans le cas d'une application différente en parfumerie, celle d'un assouplissant, la présence de 9-décen-2-one dosée à 0,5% donne de la puissance et pousse le côté fruité « pomme » et « poire » du parfum de l'assouplissant, comme indiqué dans la formule suivante :

| | |
|---|---|
| Benzyle Acétate | 70g, |
| Phenoxyallyle Acétate | 30g, |
| Alcool Phényléthylique | 80g, |
| Hexylcinnamique Aldehyde | 40g, |
| Aldéhyde C12 | 2g, |
| Ambrettolide cis-iso | 60g, |
| Aldéhyde Anisique | 30g, |
| 2-cyclohexylidene-2-phenylacetonitrile | 20g, |
| Bacdanol | 50g, |
| Citronellol | 50g, |
| Coumarine | 80g, |
| Dihydromyrcenol | 100g, |
| Acetic acid,3a,4,5,6,7,7a-hexahydro-4,7-methano-1H-indenyl ester | 65g, |
| 2-acetyl-1,2,3,4,5,6,7,8-octahydro-2,3,8,8-tetramethyl naphtalene | 106g, |
| Methylionantheme | 40g, |
| Essence de Patchouly | 30g, |
| Cyclohexyle Salicylate | 100g, |
| Dimethyl 3-cyclohexene-1-carboxaldehyde | 8g, |
| Ambroxan 5% DPG | 6g, |
| Essence de Galbanum 10% DPG | 10g, |
| Indole 10% DPG | 8g, |
| 9-decen-1-ol 10% DPG | 10g, |
| 9-décen-2-one | 5g, |
| Total | 1000 g. |

Enfin, dans le cas d'une crème, la 9-décen-2-one confère à celle-ci des notes fleuries accentuées, par exemple dans l'application suivante :

| | |
|---|---|
| Benzyle acétate | 49 g |
| Hexenyle cis-3-acétate | 2 g, |
| Linalyle acétate | 85 g, |
| Phenyléthylique alcool | 65 g, |
| Gamma undécalactone | 6 g, |
| Allyl amyl glycolate | 20 g, |
| Methyle anthranylate | 5 g, |
| Anisique aldéhyde | 10 g, |
| Methyle benzoate | 8 g, |
| Citronellol | 45 g, |
| Ethyl vanilline | 11 g, |
| Géraniol | 35 g, |
| Methyl hydrojasmonate | 130 g, |
| Heliotropine | 5 g, |
| Hexenol cis 3 | 2 g, |
| Ionone alpha | 23 g, |
| Ionone beta | 4 g, |
| 2-acetyl-1,2,3,4,5,6,7,8-octahydro-2,3,8,8-tetramethyl naphtalene | 75 g, |
| 4-(1,1-dimethylethyl)-<a>-methyl-benzenepropanal | 70g, |
| Linalol | 100 g, |
| Patchouly essence | 11 g, |
| Gamma nonalactone | 27 g, |
| Hexenyle cis-3-salicylate | 35 g, |
| Hexyle salicylate | 75 g, |
| Terpinéol | 48 g, |
| Dimethyl 3-cyclohexene-1-carboxaldehyde | 4 g, |
| Vanilline | 2 g, |
| Acétyl cédrène | 12 g, |
| Sandalore | 12 g, |
| Butyrate de diméthyl benzyl carbinol | 1 g, |
| Methyl 2,4-dihydroxy-3,6-dimethylbenzoate | 13 g, |
| 9-décen-2-one | 10 g. |

En présence de 9-décen-2-one, la crème présente plus de tête, avec des notes vertes et fraîches beaucoup plus intenses.

### Exemple 9 : Tests de toxicité

La molécule a été testée sur 3 lapins selon le protocole expérimental établi par « the OECD guideline N°404 dated April 24th 2002 » et le test « method B.4 of the Directive N°2004/73/EC) : Acute dermal irritation Il en ressort que la 9-décen-2-one n'est pas irritante et qu'aucun symbole ou phrases de risques ne sont requis pour son utilisation (selon the « EEC Directives 67/548/59 and 99/45).
De la même façon, la molécule a été testée sur 3 lapins selon le protocole expérimental établi par « the OECD guideline N°405 dated April 24th 2002 » et le test « method B.5 of the Directive N°2004/73/EC) : Acute eye irritation
Il en ressort également que la 9-décen-2-one n'est pas irritante et qu'aucun symbole ou phrases de risques ne sont requis pour son utilisation (selon the « EEC Directives 67/548/59 and 99/45).

Enfin, la valeur obtenue lors du test LD50 (acute oral toxicity (cf. : la même Directive), soit 2500 mg/kg a permis de classer la molécule comme non dangereuse.

Du fait de son innocuité, il apparaît donc que cette molécule peut être utilisable en formulation et, du fait de sa présence attestée dans la mangue et l'ananas, peut être employée dans des applications en aromatique alimentaire et en Parfumerie pouvant revendiquer un label « naturel».

## Revendications

1. Procédé de production de la 9-décen-2-one, **caractérisé par** la bioconversion de l'acide undécylénique à l'aide d'une moisissure appartenant à la famille des Mortierellaceae ou à la famille des Mucoraceae, ledit procédé comprenant les étapes suivantes:
a) la culture de ladite moisissure;
b) l'ajout du substrat acide undécylénique avec un débit de 0, 1 à 0,9 g/L/h en présence d'huile;
c) la bioconversion du substrat en 9-décen-2-one,
d) l'extraction et la purification de la 9-décen-2-one.

2. Procédé selon la revendication 1, **caractérisé en ce que** ladite moisissure appartient au genre *Mortierella,* au genre *Mucor* ou au genre *Rhizopus.*

3. Procédé selon la revendication **1 ou 2, caractérisé en ce que** l'étape de bioconversion comprend une première phase d'adaptation de la moisissure dans laquelle ledit acide undécylénique est ajouté à un débit de 0,1 à 0,5 g/L/h, puis une seconde phase dans laquelle l'acide undécylénique est ajouté à un débit de 0,25 à 0,9 g/l/h.

4. Procédé selon la revendication **3, caractérisé en ce que** la durée de ladite phase d'adaptation est inférieure à 20 heures.

5. Procédé selon la revendication **3, caractérisé en ce que** la durée de ladite phase d'adaptation est inférieure à 6 ou 12 heures.

6. Procédé selon l'une quelconque des revendications **1 à 5, caractérisé en ce que** ladite huile est choisie dans le groupe comprenant les huiles classiques alimentaires ou les triglycérides constitué d'acides gras à chaînes courtes, ou encore les huiles blanches.

7. Procédé selon l'une quelconque des revendications **1 à 6, caractérisé en ce que** ladite huile est ajoutée au milieu de fermentation à un débit compris entre 0,4 et 4,0 g/L/h.

8. Procédé selon l'une quelconque des revendications **1 à 7, caractérisé en ce que** du glucose ou du maltose est ajouté simultanément à l'acide undécylénique et à l'huile.

9. Procédé selon la revendication **8, caractérisé en ce que** ledit glucose ou ledit maltose est ajouté au milieu de fermentation à un débit inférieur à 1 g/L/h.

10. Procédé selon la revendication **8, caractérisé en ce que** ledit glucose ou ledit maltose est ajouté au milieu de fermentation à un débit inférieur à 0,75 g/L/h.

11. Procédé selon la revendication **8, caractérisé en ce que** ledit glucose ou ledit maltose est ajouté au milieu de fermentation à un débit inférieur à 0,5 g/L/h.

12. Procédé selon l'une quelconque des revendications **1 à 11, caractérisé en ce que** l'ajout dudit acide undécylénique, de ladite huile dans le milieu de fermentation se fait en continu pendant 5 à 96 heures.

13. Procédé selon l'une quelconque des revendications **8 à 11, caractérisé en ce que** l'ajout dudit acide undécylénique, de ladite huile et dudit glucose ou maltose dans le milieu de fermentation se fait en continu pendant 5 à 96 heures.

14. Procédé selon l'une quelconque des revendications **1 à 13, caractérisé en ce que** la bioconversion est réalisée à une température comprise entre 25°C et 35°C.

15. Procédé selon l'une quelconque des revendications **1 à 13, caractérisé en ce que** la bioconversion est réalisée à une température comprise entre 27 à 30°C.

16. Procédé selon l'une quelconque des revendications **1 à 15, caractérisé en ce que** le pH du milieu de fermentation est compris entre 5 et 8.

17. Procédé selon l'une quelconque des revendications **1 à 16, caractérisé en ce que** l'aération du milieu de fermentation est inférieure ou égale à 1 vvm.

18. Procédé selon l'une quelconque des revendications **1 à 17, caractérisé en ce que** la bioconversion est arrêtée par ajout dans le milieu de culture, d'un acide choisi dans le groupe comprenant l'acide phosphorique, l'acide chlorhydrique, l'acide sulfurique ou l'acide citrique.

## Patentansprüche

1. Verfahren zur Herstellung von 9-Decen-2-on, **gekennzeichnet durch** die Biokonversion von Undecylensäure mit Hilfe eines Schimmelpilzes, der zur Familie der *Mortierellaceae* oder zur Familie des *Mucoraceae* gehört, wobei das Verfahren die folgenden Schritte umfasst:
a) Kultivieren des Schimmelpilzes;
b) Zusetzen des Substrats Undecylensäure mit einem Durchsatz von 0,1 bis 0,9 g/l/h in Anwesenheit von Öl;
c) Biokonvertieren des Substrats zu 9-Decen-2-on,
d) Extrahieren und Reinigen des 9-Decen-2-on.

2. Verfahren nach Anspruch 1, **dadurch gekennzeichnet, dass** der Schimmelpilz zur Gattung *Mortierella,* zur Gattung *Mucor* oder zur Gattung *Rhizopus* gehört.

3. Verfahren nach Anspruch 1 oder 2, **dadurch gekennzeichnet, dass** der Schritt des Biokonvertierens eine erste Phase der Anpassung des Schimmelpilzes umfasst, in der die Undecylensäure mit einem Durchsatz von 0,1 bis 0,5 g/l/h zugesetzt wird, dann eine zweite Phase, in der die Undecylensäure mit einem Durchsatz von 0,25 bis 0,9 g/l/h zugesetzt wird.

4. Verfahren nach Anspruch 3, **dadurch gekennzeichnet, dass** die Dauer der Anpassungsphase weniger als 20 Stunden beträgt.

5. Verfahren nach Anspruch 3, **dadurch gekennzeichnet, dass** die Dauer der Anpassungsphase weniger als 6 oder 12 Stunden beträgt.

6. Verfahren nach einem der Ansprüche 1 bis 5, **dadurch gekennzeichnet, dass** das Öl ausgewählt ist aus der Gruppe, bestehend aus herkömmlichen Speiseölen oder Triglyceriden, die aus Fettsäuren mit kurzen Ketten bestehen, oder auch weißen Ölen.

7. Verfahren nach einem der Ansprüche 1 bis 6, **dadurch gekennzeichnet, dass** das Öl dem Fermentierungsmedium mit einem Durchsatz zugesetzt wird, der im Bereich zwischen 0,4 und 4,0 g/l/h liegt.

8. Verfahren nach einem der Ansprüche 1 bis 7, **dadurch gekennzeichnet, dass** Glucose oder Maltose gleichzeitig der Undecylensäure und dem Öl zugesetzt wird.

9. Verfahren nach Anspruch 8, **dadurch gekennzeichnet, dass** die Glucose oder die Maltose dem Fermentierungsmedium mit einem Durchsatz von weniger als 1 g/l/h zugesetzt wird.

10. Verfahren nach Anspruch 8, **dadurch gekennzeichnet, dass** die Glucose oder die Maltose dem Fermentierungsmedium mit einem Durchsatz von weniger als 0,75 g/l/h zugesetzt wird.

11. Verfahren nach Anspruch 8, **dadurch gekennzeichnet, dass** die Glucose oder die Maltose dem Fermentierungsmedium mit einem Durchsatz von weniger als 0,5 g/l/h zugesetzt wird.

12. Verfahren nach einem der Ansprüche 1 bis 11, **dadurch gekennzeichnet, dass** der Zusatz der Undecylensäure, des Öls in das Fermentierungsmedium ununterbrochen während 5 bis 96 Stunden erfolgt.

13. Verfahren nach einem der Ansprüche 8 bis 11, **dadurch gekennzeichnet, dass** der Zusatz der Undecylensäure, des Öls und der Glucose oder Maltose in das Fermentierungsmedium ununterbrochen während 5 bis 96 Stunden erfolgt.

14. Verfahren nach einem der Ansprüche 1 bis 13, **dadurch gekennzeichnet, dass** die Biokonversion bei einer Temperatur durchgeführt wird, die im Bereich zwischen 25 °C und 35 °C liegt.

15. Verfahren nach einem der Ansprüche 1 bis 13, **dadurch gekennzeichnet, dass** die Biokonversion bei einer Temperatur durchgeführt wird, die im Bereich zwischen 27 °C und 30 °C liegt.

16. Verfahren nach einem der Ansprüche 1 bis 15, **dadurch gekennzeichnet, dass** der pH-Wert des Fermentierungsmediums zwischen 5 und 8 liegt.

17. Verfahren nach einem der Ansprüche 1 bis 16, **dadurch gekennzeichnet, dass** die Belüftung des Fermentierungsmediums kleiner oder gleich 1 vvm ist.

18. Verfahren nach einem der Ansprüche 1 bis 17, **dadurch gekennzeichnet, dass** die Biokonversion durch den Zusatz in das Kulturmedium einer Säure gestoppt wird, ausgewählt aus den Gruppe, bestehend aus Phosphorsäure, Chlorwasserstoffsäure, Schwefelsäure oder Zitronensäure.

## Claims

1. Method for producing 9-decen-2-one, **characterised by** the bioconverting of undecylenic acid using a mould belonging to the family Mortierellaceae or to the family Mucoraceae, said method comprising the following steps:
a) the culturing of said mould;
b) the adding of the undecylenic acid substrate with a flow rate from 0.1 to 0.9 g/L/h in the presence of oil;
c) the bioconverting of the substrate into 9-decen-2-one,
d) the extracting and the purifying of the 9-decen-2-one.

2. Method according to claim 1, **characterised in that** said mould belongs to the genus Mortierella, to the genus Mucor or to the genus Rhizopus.

3. Method according to claim 1 or 2, **characterised in that** the step of bioconverting comprises a first phase of adapting the mould wherein said undecylenic acid is added at a flow rate from 0.1 to 0.5 g/L/h, then a second phase wherein undecylenic acid is added at a flow rate from 0.25 to 0.9 g/l/h.

4. Method according to claim 3, **characterised in that** the duration of said phase of adaptation is less than 20 hours.

5. Method according to claim 3, **characterised in that** the duration of said phase of adaptation is less than 6 or 12 hours.

6. Method according to any of claims 1 to 5, **characterised in that** said oil is chosen from the group comprising conventional edible oils or triglycerides formed from short-chain fatty acids, or white oils.

7. Method according to any of claims 1 to 6, **characterised in that** said oil is added to the fermentation medium at a flow rate between 0.4 and 4.0 g/L/h.

8. Method according to any of claims 1 to 7, **characterised in that** glucose or maltose is added simultaneously to the undecylenic acid and to the oil.

9. Method according to claim 8, **characterised in that** said glucose or said maltose is added to the fermentation medium at a flow rate less than 1 g/L/h.

10. Method according to claim 8, **characterised in that** said glucose or said maltose is added to the fermentation medium at a flow rate less than 0.75 g/L/h.

11. Method according to claim 8, **characterised in that** said glucose or said maltose is added to the fermentation medium at a flow rate less than 0.5 g/L/h.

12. Method according to any of claims 1 to 11, **characterised in that** the adding of said undecylenic acid, of said oil in the fermentation medium is carried out continuously for 5 to 96 hours.

13. Method according to any of claims 8 to 11, **characterised in that** the adding of said undecylenic acid, of said oil and of said glucose or maltose in the fermentation medium is carried out continuously for 5 to 96 hours.

14. Method according to any of claims 1 to 13, **characterised in that** the bioconverting is carried out at a temperature between 25°C and 35°C.

15. Method according to any of claims 1 to 13, **characterised in that** the bioconverting is carried out at a temperature between 27 to 30°C.

16. Method according to any of claims 1 to 15, **characterised in that** the pH of the fermentation medium is between 5 and 8.

17. Method according to any of claims 1 to 16, **characterised in that** the aeration of the fermentation medium is less than or equal to 1 vvm.

18. Method according to any of claims 1 to 17, **characterised in that** the bioconverting is stopped by adding to the culture medium, an acid chosen from the group comprising phosphoric acid, hydrochloric acid, sulphuric acid or citric acid.
